# FASCICULE DE BREVET EUROPEEN

(11) **EP 0 998 326 B1**
(45) Date de publication et mention de la délivrance du brevet: **17.10.2001**
(21) Numéro de dépôt: 98940322.5
(22) Date de dépôt: 23.07.1998
(51) Int. Cl.: A61N 1/32, A61N 1/04

(54) **DISPOSITIF DE TRAITEMENT THERAPEUTIQUE DE PLAIES**
VORRICHTUNG ZUR THERAPEUTISCHEN BEHANDLUNG VON WUNDEN
DEVICE FOR THERAPEUTIC TREATMENT OF WOUNDS

(30) Priorité: 25.07.1997 FR 9709506
(43) Date de publication de la demande: 10.05.2000
(73) Titulaire: LABORATOIRES D'HYGIENE ET DE DIETETIQUE (L.H.D.), 75008 Paris (FR)
(72) Inventeur: MILLOT, Philippe, Pierre, Marie, F-21490 Orgeux (FR); LAMOISE, Michel, F-21110 Bessey-les-Citeaux (FR)
(74) Mandataire: Colas, Jean-Pierre
(86) Numéro de dépôt international: FR9801632
(87) Numéro de publication internationale: WO9904852

(56) Documents cités:
- EP-A- 0 367 320
- EP-A- 0 504 715
- US-A- 4 895 154
- US-A- 4 982 742
- US-A- 5 395 398

## Description

La présente invention est relative à un dispositif de traitement thérapeutique de plaies et, plus particulièrement, à un tel dispositif du type comprenant un pansement muni d'une couche d'absorption des exsudats de la plaie, et des moyens d'alimentation électrique pour faire circuler un courant dans ladite plaie, à travers ledit pansement.

De nombreux types de pansements ont été conçus pour absorber les exsudats d'une plaie et accélérer la cicatrisation de cette plaie. A cet égard, on connaît en particulier un pansement adhésif semi-occlusif et stérile constitué par une couche de masse hydrocolloïde appliquée sur un support constitué d'un film en polyuréthanne pour le traitement local de plaies telles qu'ulcères de jambes ou escarres. Les laboratoires URGO de Chenôve (France) fabriquent un tel pansement, commercialisé sous le nom ALGOPLAQUE (marque déposée). Au contact d'une plaie, les particules hydrocolloïdes dispersées dans la couche absorbent les exsudats, se gonflent et forment un gel humide favorisant la cicatrisation de la plaie sans que les tissus nouvellement formés ne soient endommagés lors du renouvellement du pansement.

L'électrothérapie des plaies est aussi une technique connue pour accélérer leur cicatrisation. Cette technique consiste généralement à appliquer deux électrodes sur la peau d'un patient et à faire passer un courant électrique entre ces électrodes de manière que celui-ci passe dans une plaie de cette peau. On décrit en particulier dans le document EP-A-0 367 320 un système de traitement de plaie comprenant un pansement constitué par une compresse conductrice de l'électricité, une électrode de retour et des moyens pour faire passer un courant électrique entre la compresse et l'électrode de retour. La compresse comprend une couche d'un gel hydrophile conducteur de l'électricité, pouvant contenir jusqu'à 93-96 % d'eau liée, et rendu conducteur par un sel métallique. La couche de gel est accolée à une couche conductrice d'électricité. Un courant électrique passe dans cette couche et dans la couche de gel hydrophile puis dans la plaie avant de rejoindre l'électrode de retour. On comprend que la capacité d'absorption des exsudats de la plaie par un tel gel est limitée par son humidité initiale.

La présente invention a pour but de réaliser un dispositif de traitement thérapeutique de plaies, dans lequel la capacité d'absorption des exsudats de la plaie est maximisée tout en étant combinée à un traitement électrique de cette plaie.

On atteint ce but de l'invention, ainsi que d'autres qui apparaîtront à la lecture de la description qui va suivre, avec un dispositif de traitement thérapeutique de plaies qui comprend un pansement muni d'une couche d'absorption des exsudats de la plaie et des moyens d'alimentation électrique pour faire circuler un courant dans ladite plaie, à travers ledit pansement, ce dispositif étant remarquable en ce que ladite couche est une couche hydrophile sèche et sensiblement non conductrice de l'électricité au moment de son application sur la plaie, la migration ultérieure des exsudats dans l'épaisseur de ladite couche commandant l'activation desdits moyens d'alimentation électrique.

On comprend que ladite couche hydrophile initialement sèche est ainsi utilisée sans altération de sa capacité d'absorption des exsudats. Comme on le verra plus loin, cette couche, initialement non conductrice, devient conductrice au cours de son chargement en exsudats. Elle devient alors, à un certain moment, suffisamment conductrice pour permettre un traitement "électrique" de la plaie.

A cet égard, suivant une caractéristique du dispositif selon la présente invention, les moyens d'alimentation électrique comprennent un circuit de mesure de l'impédance de la couche hydrophile et des moyens sensibles au passage de ladite mesure en dessous d'un seuil prédéterminé pour commander l'activation des moyens d'alimentation électrique en régime nominal.

D'autres caractéristiques et avantages de la présente invention apparaîtront à la lecture de la description qui va suivre et à l'examen du dessin annexé dans lequel :
- les figures 1 et 2 sont des vues en plan et en coupe, respectivement, d'un premier mode de réalisation d'un pansement qui comprend à la fois une électrode conductrice dite "de retour" et l'électrode en contact avec la plaie, dite "active", formant partie du dispositif suivant l'invention,
- la figure 3 est une vue en coupe d'un premier mode de réalisation du dispositif suivant l'invention, constitué par le pansement de la figure 1 et 2 et de moyens d'alimentation électrique, intégrés au pansement,
- la figure 4 représente un deuxième mode de réalisation d'un pansement pour le dispositif suivant l'invention,
- la figure 5 représente un autre mode de réalisation d'un pansement pour le dispositif suivant l'invention dans lequel seule l'électrode dite "active" est incluse dans le pansement, l'électrode dite "de retour" étant indépendante,
- la figure 6 représente un deuxième mode de réalisation des moyens d'alimentation électrique du dispositif suivant l'invention, conçus pour être associés au pansement de la figure 5,
- la figure 7 est un schéma fonctionnel des moyens d'alimentation électrique du dispositif suivant l'invention, et
- les figures 8 et 9 sont des graphes illustrant les processus de chargement en exsudats et d'évolution de l'impédance, respectivement, d'une couche hydrophile formant partie du pansement suivant l'invention.

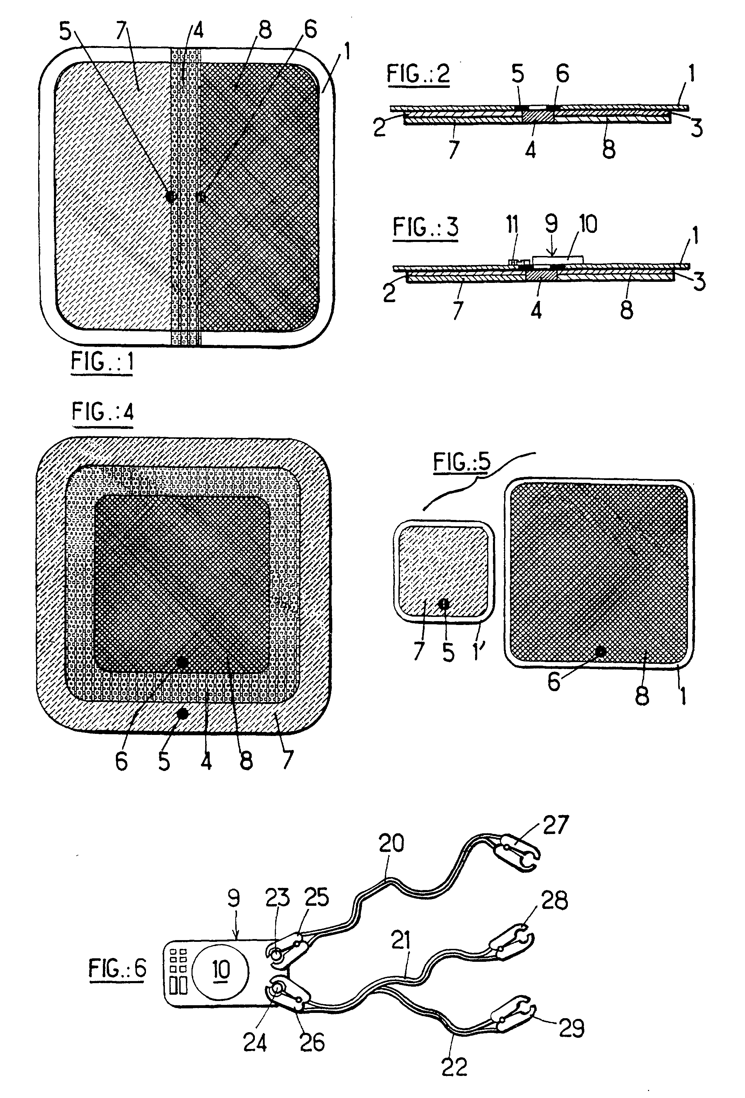

On se réfère aux figures 1 et 2 du dessin annexé où il apparaît que le premier mode de réalisation du pansement formant partie du dispositif suivant l'invention prend une forme sensiblement plane et rectangulaire, de quelques centimètres de côté par exemple, de manière à présenter une surface sensiblement double de celle d'une plaie à recouvrir.

Le pansement comprend, sur un support 1 souple, deux électrodes 2 et 3 séparées par une bande 4 en un matériau isolant. Les électrodes 2 et 3 sont en contact électrique avec deux plots 5,6 respectivement, en matériau conducteur de l'électricité, ces plots traversant le support 1 pour affleurer à sa surface extérieure.

L'une des électrodes, l'électrode 2 par exemple, dite électrode de retour, est recouverte d'une couche conductrice d'interface 7 avec la peau du patient, alors que l'autre électrode 3 dite électrode active, qui est en contact avec la plaie est recouverte, selon l'invention, d'une couche 8 hydrophile sèche, non conductrice de l'électricité à l'état sec.

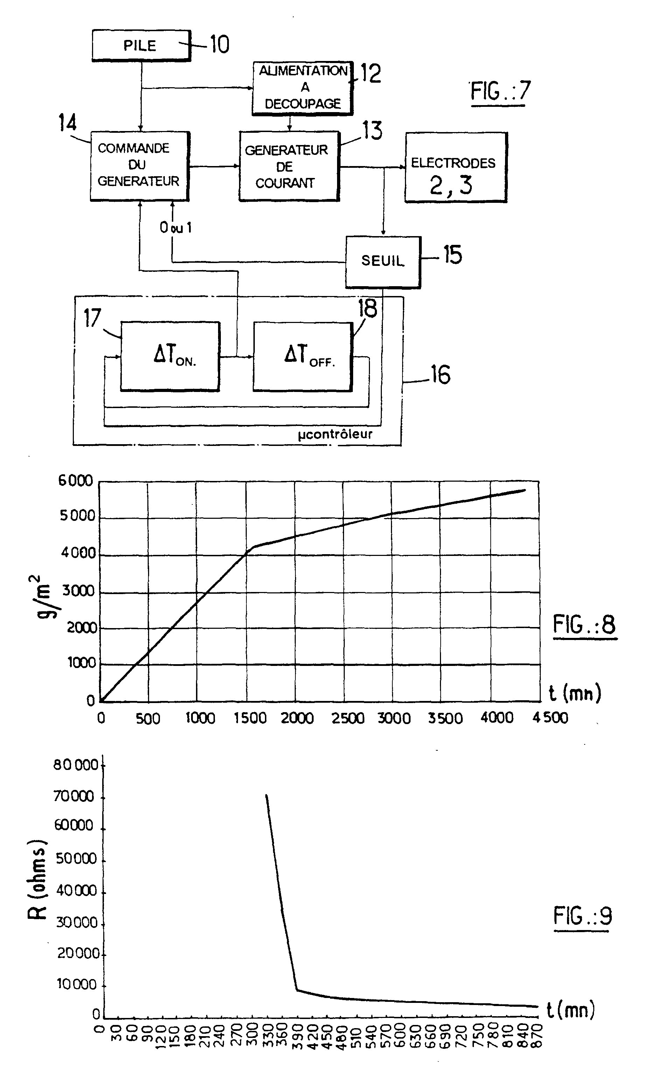

Des moyens d'alimentation électrique, décrits plus loin en détail en liaison avec la figure 7, sont connectés aux plots 5,6 pour établir et commander un courant électrique entre les deux électrodes, quand le pansement est appliqué contre la peau d'un patient, la couche 8 hydrophile sèche étant appliquée contre une plaie à cicatriser alors que la couche conductrice 7 est appliquée sur une plage de la peau adjacente à cette plaie.

Pour fixer le pansement dans cette position, des moyens adhésifs (non représentés) peuvent être prévus sur le pansement, par exemple sur le support 1, au niveau de la périphérie de celui-ci.

Les moyens d'alimentation électrique peuvent être physiquement séparés du pansement et raccordés aux plots 5,6 par des fils électriques. Ils peuvent aussi être intégrés au pansement et jetables avec lui. On a représenté à la figure 3 de tels moyens 9 comportant une source d'énergie électrique autonome telle qu'une pile 10 et divers composants électroniques 11, discrets ou intégrés. Ces moyens 9 sont fixés sur le support souple 1, au-dessus des plots 5,6. En variante, ces moyens peuvent être reçus amoviblement sur le pansement, grâce à des moyens mécaniques d'assemblage tels que boutons-pression, rainures, grilles, fermetures "Velcro" (marque déposée), etc... de manière à pouvoir en être retirés après traitement puis réutilisés sur un pansement neuf.

Dès le pansement fixé sur la plaie, la couche hydrophile 8 sèche commence à se charger en exsudats venus de la plaie. Des essais in vitro ont été conduits pour préciser ce processus. On a utilisé des cellules remplies de sérum physiologique et fermées par un échantillon d'une couche 8 constituée d'une masse hydrocolloïde de 1 mm d'épaisseur en contact avec le sérum, ce dernier simulant les exsudats d'une plaie. La cinétique de chargement de la couche en sérum physiologique a été établie par le relevé et le cumul, dans le temps, de la quantité de sérum absorbée par la couche entre deux relevés consécutifs. Le graphe dé la figure 8 représente la cinétique de chargement moyenne des cellules utilisées.

Par ailleurs, l'évolution de la résistance de la couche a été relevée par l'établissement, à des intervalles de temps réguliers, d'un courant électrique dans la cellule, à travers la couche, et la mesure de la tension résultante, la densité de courant établie étant alors de 0,11 mA/cm² pendant les temps de mesure. Le graphe de la figure 9 représente l'évolution dans le temps de la moyenne des résistances ainsi calculées sur les cellules utilisées. Comme le montre ce graphe, pendant les premières heures, aucune évaluation de la conductivité n'est possible du fait que la couche 8 reste alors sensiblement non conductrice de l'électricité.

On peut expliquer la brusque apparition d'une conductivité dans la couche 8, illustrée par la figure 9 et observée au bout de 6 à 8 heures, par une migration progressive régulière du sérum, et donc des exsudats d'une plaie, riches en sels minéraux et donc fortement conducteurs, depuis la face de la couche 8 qui est en contact avec la plaie jusqu'à l'autre face de cette couche, en contact avec l'électrode 3. Aussi longtemps que ces exsudats n'ont pas atteint cette autre face, la résistance de la couche 8 reste élevée. Elle tombe à une valeur beaucoup plus basse dès que les exsudats atteignent cette autre face. La couche 8 étant alors conductrice dans toute son épaisseur, elle est propre à être traversée par un courant électrique, dans le cas d'un traitement visant à accélérer la cicatrisation de la plaie.

Ainsi, suivant l'invention, on parvient à combiner les effets bénéfiques d'une absorption des exsudats de la plaie dans une couche hydrophile et un tel traitement électrique, sans sacrifier en rien la capacité d'absorption de la couche hydrophile utilisée qui devait, dans la technique antérieure, être initialement rendue conductrice par une charge d'eau et de sels minéraux, au détriment de sa capacité totale d'absorption des exsudats.

A cet égard, il apparaît sur le graphe de la figure 8 qu'au bout de sept heures environ (420 minutes), la couche hydrophile 8 s'est chargée de 1000 g/m² environ d'exsudats pour atteindre une conductivité compatible avec un traitement électrique de la plaie, cette charge se substituant avantageusement au chargement en solution saline pratiqué dans la technique antérieure pour lui donner cette conductivité.

Dans la présente invention, le traitement électrique est retardé de quelques heures ce qui est peu de chose, comparé au temps de cicatrisation d'une plaie, couramment de l'ordre de plusieurs jours à plusieurs semaines. En outre, l'absorption des exsudats par la couche hydrophile démarre dès la mise en place du pansement, avant le traitement électrique de la plaie.

De nombreux produits hydrophiles secs, seuls ou en mélange, par exemple sous forme de fibres, de particules dispersées ou de mousse peuvent être utilisés pour constituer la couche 8 du pansement selon la présente invention. L'épaisseur de la couche est avantageusement comprise entre 200 et 2000 µm, de préférence entre 300 et 1000 µm. Si la couche n'est pas naturellement autoadhésive, le pansement peut être muni de moyens adhésifs assurant la fixation du pansement sur la peau du patient, comme on l'a vu plus haut.

La couche hydrophile sèche peut être réalisée à partir de tout matériau ou compositions absorbantes hydrophiles sèches employés dans la réalisation de pansements destinés à traiter les plaies exsudatives.

Elle peut ainsi se présenter sous forme de mousses absorbantes, en particulier des mousses de polyuréthanne hydrophiles employées dans les pansements dits hydrocellulaires, sous forme de fibres à base de matériaux absorbants comme par exemple des fibres d'alginate, tels les alginates de sodium ou de calcium, ou des fibres de dérivés cellulosiques, sous forme de compresses en non-tissé, sous forme de gels lyophilisés et sous forme de compositions hydrocolloïdales telles des gels ou des masses hydrocolloïdales telles celles employées dans les pansements du type dit hydrocolloïdes.

Dans le cadre de la présente invention, on préférera comme couche hydrophile sèche les masses hydrocolloïdes.

Ces masses hydrocolloïdes sont formées principalement d'un élastomère adhésif choisi parmi les polymères tels que les polyisobutènes ou des copolymères séquencés poly(styrène-oléfines-styrène) comme par exemple poly(styrène-isoprène-styrène) ou poly(styrène-éthylène butylène-styrène), associé ou non à des résines poisseuses dites "tackifiantes", des plastifiants etc... et d'un ou plusieurs hydrocolloïdes. Par hydrocolloïdes utilisables dans ces masses, on entend désigner ici les composés couramment utilisés par l'homme de l'art, connus pour leur aptitude à absorber les liquides hydrophiles, en particulier l'eau et les exsudats et les transporter rapidement. Comme hydrocolloïdes appropriés, on peut citer par exemple l'alcool polyvinylique, la gélatine, la pectine, les gommes végétales naturelles telles que la gomme de caroube, la gomme de Karaya, la gomme guar, la gomme arabique..., les dérivés de cellulose tels que les hydroxyéthylcelluloses, les hydroxypropylcelluloses, les carboxyméthylcelluloses et leurs sels de métal alcalin, tel le sodium ou le calcium. On pourra utiliser ces hydrocolloides seuls ou en association.

De telles masses sont ainsi décrites dans les demandes de brevet EP-A-130 061, EP-092 999, WO-98/10801 ainsi que dans le brevet US 3 972 328.

Le support souple 1 peut être découpé dans un film en matière plastique tel que du polyéthylène, du polyuréthanne, du polyester ou une polyimide. Les électrodes 2 et 3 peuvent être formées sur ce film par sérigraphie d'une encre conductrice. On pourra utiliser une encre du type Ag/AgCl pour constituer des électrodes dites "sacrificielles" propres à éviter une électrolyse de l'eau et donc propres à maintenir constant le pH des exsudats au niveau de la couche 8 de la plaie, si besoin est.

Les électrodes 2,3 peuvent aussi être métalliques, en cuivre, zinc, argent, or, platine par exemple, ou bien être en carbone ou encore en un polymère conducteur.

La bande 4 est réalisée en un matériau propre à constituer une barrière aussi bien électriquement que vis-à-vis de l'humidité, tel qu'une mousse de matière plastique.

L'électrode 2, dite "de retour", est recouverte d'une couche 7 d'un matériau assurant un bon contact avec la peau et le retour du courant. Cette couche pourra être constituée de polyoxyéthylène ou de polyéthylène-glycol par exemple, réticulé et présentant une teneur en eau et en sels suffisante pour être bonne conductrice de l'électricité.

On se réfère maintenant à la figure 7 du dessin annexé pour décrire la structure d'un mode de réalisation des moyens d'alimentation électrique du dispositif suivant l'invention. Quand ces moyens sont intégrés au pansement, comme on l'a vu plus haut, ils prennent la forme d'un circuit électronique dont les composants, pile, composants discrets ou intégrés, sont reportés sur des pistes conductrices formées sur le support 1, par toute technique bien connue : refusion, collage, soudure à la vague, etc... Ils peuvent aussi être amovibles et réutilisables, voire entièrement distincts du pansement auquel ils sont raccordés alors par des liaisons électriques convenables.

Dans le mode de réalisation de ce circuit schématisé à la figure 7, le circuit comprend essentiellement une ou plusieurs piles électriques 10, par exemple du type "bouton", propres à assurer une alimentation électrique autonome du circuit, avec de faibles poids et encombrement compatibles avec le caractère portable du pansement sur lequel il est monté, au moins dans le cas du mode de réalisation de ce pansement représenté à la figure 3. La pile 10 alimente une alimentation à découpage 12 qui relève la tension disponible aux bornes de la pile, pour assurer l'alimentation électrique d'un générateur de courant 13 de tout type convenable, par exemple du type Howlland. Ce générateur débite du courant dans les électrodes 2,3, sous la commande d'un sous-circuit 14.

Suivant la présente invention, la couche 8 hydrophile sèche présente, au début d'un traitement, une impédance sensiblement infinie, du fait qu'elle n'est pas encore hydratée par les exsudats de la plaie. Il convient, pour commander le passage d'un courant électrique convenable, d'une intensité de l'ordre du milliampère par exemple, d'attendre que l'hydratation de la couche ait amené son impédance à une valeur suffisamment basse, de l'ordre de quelques kΩ par exemple.

Dans un premier temps donc, au début d'un traitement, le sous-circuit 14 commande un générateur 13 de manière que celui-ci débite, ou tente de débiter, entre les électrodes un courant de faible intensité, propre seulement à permettre la mesure de l'impédance de la couche 8 par le sous-circuit 15. Celui-ci compare l'impédance mesurée à un seuil prédéterminé, permettant de détecter la chute brutale d'impédance qui apparaît sur le graphe de la figure 9. Lorsque ce seuil est atteint, au bout de six à huit heures par exemple, le sous-circuit 14 commute le courant débité par le générateur 13 à une intensité nominale prédéterminée, correspondant par exemple à la saturation de ce générateur. Le traitement électrique de la plaie peut alors commencer.

Celui-ci peut se développer dans le temps suivant un programme prédéterminé, alternant des intervalles de temps **ΔT**_{**ON**} avec passage de courant dans la plaie et des intervalles ΔT_{**OFF**} sans passage de courant dans cette plaie. La programmation et la commande de la succession de ces intervalles de temps peut être assurée par un microcontrôleur 16 et par des compteurs de temps 17,18 internes à ce microcontrôleur, comme représenté, ou externes à celui-ci.

Il apparaît maintenant que l'invention permet bien d'atteindre les buts fixés, à savoir fournir un dispositif de traitement d'une plaie par électrothérapie comportant une couche d'absorption des exsudats de la plaie dont la capacité d'absorption de ces exsudats n'est pas entamée par la nécessité de rendre cette couche conductrice de l'électricité. On remarquera que, dans le mode de réalisation de ce dispositif représenté à la figure 3, celui-ci prend une forme particulièrement compacte, semblable à celle d'un pansement classique, les moyens de traitement par électrothérapie étant intégrés au pansement.

Bien entendu, l'invention n'est pas limitée aux modes de réalisation représentés aux figures 1 à 3. C'est ainsi que les électrodes du dispositif, qui sont adjacentes dans le mode de réalisation des figures 1 à 3, pourraient aussi être concentriques, comme représenté à la figure 4 où des références identiques à des références utilisées sur la figure 1 repèrent des éléments ou organes identiques ou similaires. La couche hydrophile sèche 8 est alors placée au centre et la couche conductrice 7 placée sur l'électrode de retour 2 présente un contour fermé, cette couche étant séparée de la couche 8 par une bande isolante 4, également de contour fermé.

Le pansement peut comprendre uniquement l'électrode (3) dite électrode active comme dans le mode de réalisation du dispositif suivant l'invention représenté à la figure 5. Dans ce cas, l'électrode (2 dite de retour est formée sur un support distinct (1') du support (1) de l'électrode active (2) du pansement et est totalement indépendante du pansement. Pour l'alimentation électrique du pansement, il convient alors de disposer des moyens représentés à la figure 6 comprenant un circuit 9 du type décrit en liaison avec la description du mode de réalisation des figures 1 à 3 et des lignes électriques souples telles que 20,21,22 permettant de raccorder les pôles 23,24 du circuit aux plots de contact 5,6 des électrodes du mode de réalisation de la figure 5, par l'intermédiaire de pinces 25,26,27,28,29. On notera que les pinces 28,29 permettent de raccorder deux électrodes de retour au circuit 9. On peut évidemment faire varier le nombre de ces électrodes de retour, installées à distance de l'électrode qui est appliquée sur la plaie à traiter, pour assurer une distribution plus régulière du courant thérapeutique dans cette plaie.

## Revendications

1. Dispositif de traitement thérapeutique de plaies, qui comprend un pansement muni d'une couche (8) d'absorption des exsudats de la plaie et des moyens d'alimentation électrique (9) pour faire circuler un courant dans ladite plaie, à travers ledit pansement, **caractérisé en ce que** ladite couche (8) est une couche hydrophile sèche et sensiblement non conductrice de l'électricité au moment de son application sur la plaie, la migration ultérieure des exsudats dans l'épaisseur de ladite couche (8) commandant l'activation desdits moyens d'alimentation électrique (9), parallèlement à la poursuite du processus d'absorption des exsudats par ladite couche.

2. Dispositif conforme à la revendication 1, **caractérisé en ce que** ladite couche hydrophile sèche est choisie dans le groupe constitué par : les mousses de polyuréthanne, les compresses en non-tissé, les masses ou les gels hydrocolloïdes, les fibres d'alginates ou les dérivés cellulosiques sous forme de gels lyophilisés.

3. Dispositif conforme à l'une quelconque des revendications 1 et 2, **caractérisé en ce que** ledit pansement comprend deux électrodes (2,3) formées sur un même support plan (1), l'une des électrodes étant recouverte de ladite couche hydrophile sèche, lesdits moyens d'alimentation (9) étant connectés auxdites électrodes (2,3) pour faire circuler un courant électrique entre elles.

4. Dispositif conforme à la revendication 3, **caractérisé en ce que** lesdites électrodes (2,3) sont adjacentes.

5. Dispositif conforme à la revendication 3, **caractérisé en ce que** lesdites électrodes (2,3) sont concentriques.

6. Dispositif conforme à l'une quelconque des revendications 1 et 2, **caractérisé en ce qu'**il comprend a) un pansement, disposé au contact de la plaie, constitué d'une électrode (3) dite électrode active formée sur un support plan (1), ladite électrode (3) étant recouverte de ladite couche (8) hydrophile sèche, et b) au moins une autre électrode (2), dite électrode de retour formée sur un support plan (1') distinct de celui de l'électrode dite active, lesdits moyens d'alimentation étant connectés auxdites électrodes (2,3) pour faire circuler un courant électrique entre elles.

7. Dispositif conforme à l'une quelconque des revendications 1 à 6, **caractérisé en ce que** ledit pansement comprend des moyens adhésifs permettant sa fixation sur la peau d'un patient.

8. Dispositif conforme à l'une quelconque des revendications précédente, **caractérisé en ce que** lesdits moyens d'alimentation électrique (9) comprennent des moyens de commande (14) de l'intensité du courant débité.

9. Dispositif conforme à la revendication 8, **caractérisé en ce que** lesdits moyens d'alimentation électrique comprennent des moyens (16,17,18) de commande dans le temps du courant débité.

10. Dispositif conforme à l'une quelconque des revendications 8 et 9, **caractérisé en ce que** lesdits moyens d'alimentation (9) comprennent un circuit de mesure (15) de l'impédance de la couche hydrophile et des moyens (14) sensibles au passage de ladite mesure en dessous d'un seuil prédéterminé pour commander l'activation des moyens d'alimentation (9) en régime nominal.

11. Dispositif conforme à l'une quelconque des revendications 1 à 10, **caractérisé en ce que** lesdits moyens d'alimentation (9) sont intégrés au pansement.

12. Dispositif conforme à l'une quelconque des revendications 1 à 10, **caractérisé en ce que** lesdits moyens d'alimentation (9) sont conçus pour être montés amoviblement sur le pansement.

13. Pansement utilisable dans la réalisation d'un dispositif de traitement thérapeutique de plaie, constitué d'une électrode (3) formée sur un support plan (1), **caractérisé en ce que** ladite électrode (3) est recouverte d'une couche (8) hydrophile sèche non conductrice de l'électricité au moment de son application sur la plaie.

14. Pansement conforme à la revendication 13 **caractérisé en ce que** la couche (8) hydrophile sèche est une masse hydrocolloïde.

## Patentansprüche

1. Vorrichtung zur therapeutischen Behandlung von Wunden, die einen Verband, der mit einer Schicht (8) zur Absorption der Wundexsudate versehen ist, und Mittel (9) zur elektrischen Versorgung aufweist, um durch den Verband hindurch in der Wunde einen Strom fließen zu lassen, **dadurch gekennzeichnet, dass** die Schicht (8) eine trockene hydrophile Schicht ist, die zum Zeitpunkt ihres Auflegens auf die Wunde im wesentlichen nicht elektrisch leitend ist, wobei das nachfolgende Einsickern der Exsudate in die Dicke dieser Schicht (8) die Aktivierung der Mittel (9) zur elektrischen Versorgung auslöst, und zwar parallel zum weiteren Ablauf des Prozesses der Absorption der Exsudate durch diese Schicht.

2. Vorrichtung nach Anspruch 1, **dadurch gekennzeichnet, dass** die trockene hydrophile Schicht aus der Gruppe ausgewählt ist, die besteht aus: Polyurethanschäumen, Faservlieskompressen, Hydrokolloidmassen oder -gelen, Alginatfasern oder Cellulosederivaten in Form von lyophilisierten Gelen.

3. Vorrichtung nach einem der Ansprüche 1 und 2, **dadurch gekennzeichnet, dass** der Verband zwei auf einem gemeinsamen ebenen Träger (1) gebildete Elektroden (2, 3) umfasst, deren eine mit der trockenen hydrophilen Schicht bedeckt ist, wobei die Versorgungsmittel (9) an diese Elektroden (2, 3) angeschlossen sind, um zwischen ihnen einen elektrischen Strom fließen zu lassen.

4. , Vorrichtung nach Anspruch 3, **dadurch gekennzeichnet, dass** die Elektroden (2, 3) nebeneinander angeordnet sind.

5. Vorrichtung nach Anspruch 3, **dadurch gekennzeichnet, dass** die Elektroden (2, 3) konzentrisch sind.

6. Vorrichtung nach einem der Ansprüche 1 und 2, **dadurch gekennzeichnet, dass** sie umfasst:
a) einen im Kontakt mit der Wunde angeordneten Verband, der aus einer auf einem ebenen Träger (1) gebildeten Elektrode (3), aktive Elektrode genannt, besteht, die mit der trockenen hydrophilen Schicht (8) bedeckt ist, und
b) mindestens eine weitere Elektrode (2), Rückelektrode genannt, die auf einem vom Träger der sog. aktiven Elektrode getrennten ebenen Träger (1') gebildet ist, wobei die Versorgungsmittel an diese Elektroden (2, 3) angeschlossen sind, um zwischen ihnen einen elektrischen Strom fließen zu lassen.

7. Vorrichtung nach einem der Ansprüche 1 bis 6, **dadurch gekennzeichnet, dass** der Verband Klebemittel aufweist, die seine Befestigung auf der Haut eines Patienten gestatten.

8. Vorrichtung nach einem der vorhergehenden Ansprüche, **dadurch gekennzeichnet, dass** die Mittel (9) zur elektrischen Versorgung Mittel (14) zur Steuerung der Stromstärke des abgegebenen Stroms umfassen.

9. Vorrichtung nach Anspruch 8, **dadurch gekennzeichnet, dass** die Mittel zur elektrischen Versorgung Mittel (16, 17, 18) zur zeitlichen Steuerung des abgegebenen Stroms umfassen.

10. Vorrichtung nach einem der Ansprüche 8 und 9, **dadurch gekennzeichnet, dass** die Versorgungsmittel (9) eine Schaltung (15) zur Messung der Impedanz der hydrophilen Schicht und Mittel (14) umfassen, die gegenüber dem Abfallen der Messung unter eine vorbestimmte Schwelle empfindlich sind, um die Aktivierung der Versorgungsmittel (9) im Nennbetrieb auszulösen.

11. Vorrichtung nach einem der Ansprüche 1 bis 10, **dadurch gekennzeichnet, dass** die Versorgungsmittel (9) in den Verband integriert sind.

12. Vorrichtung nach einem der Ansprüche 1 bis 10, **dadurch gekennzeichnet, dass** die Versorgungsmittel (9) dafür ausgelegt sind, auf dem Verband lösbar montiert zu werden.

13. Verband, der bei der Herstellung einer Vorrichtung zur therapeutischen Behandlung von Wunden verwendbar ist, die aus einer auf einem ebenen Träger (1) gebildeten Elektrode (3) besteht, , **dadurch gekennzeichnet, dass** die Elektrode (3) mit einer trockenen hydrophilen Schicht (8) bedeckt ist, die zum Zeitpunkt ihres Auflegens auf die Wunde nicht elektrisch leitend ist.

14. Verband nach Anspruch 13, **dadurch gekennzeichnet, dass** die trockene hydrophile Schicht (8) eine Hydrokolloidmasse ist.

## Claims

1. Device for therapeutic treatment of wounds, which includes a dressing provided with a layer (8) for absorbing exudates from the wound and electric supply means (9) to circulate a current in said wound, through said dressing, **characterised in that** said layer (8) consists of a dry hydrophile layer which is substantially electrically non-conductive at the time it is applied on the wound, subsequent migration of exudates into the thickness of said layer (8) controlling the activation of said electric supply means (9), concomitantly with continuation of the process of exudate absorption by said layer.

2. Device according to claim 1, **characterised in that** said dry hydrophile layer is chosen from the group including: polyurethane foams, non-woven compresses, hydrocolloid masses or gels, fibres of alginates or cellulose derivatives in the form of freeze-dried gels.

3. Device according to claim 1 or claim 2, **characterised in that** said dressing includes two electrodes (2, 3) formed on a common plane support (1), one of said electrodes being covered with said dry hydrophile layer, said supply means (9) being connected to said electrodes (2, 3) to cause an electric current to flow between them.

4. Device according to claim 3, **characterised in that** said electrodes (2, 3) are adjacent.

5. Device according to claim 3, **characterised in that** said electrodes (2, 3) are concentric.

6. Device according to claim 1 or claim 2, **characterised in that** it includes a) a dressing disposed in contact with the wound consisting of an active electrode (3) formed on a plane support (1), said electrode (3) being covered with said dry hydrophile layer (8), and b) at least one return electrode (2) formed on a plane support (1') separate from that of the active electrode, said supply means being connected to said electrodes (2, 3) to cause an electric current to flow between them.

7. Device according to any of claims 1 to 6, **characterised in that** said dressing includes adhesive means enabling it to be fixed to the skin of a patient.

8. Device according to any preceding claim, **characterised in that** said electric supply means (9) include means (14) for controlling the output current.

9. Device according to claim 8, **characterised in that** said electric supply means include means (16, 17, 18) for controlling the output current in time.

10. Device according to claim 8 or claim 9, **characterised in that** said supply means (9) include a circuit (15) for measuring the impedance of the hydrophile layer and means (14) responsive to said measurement falling below a predetermined threshold to command the activation of the supply means (9) under nominal conditions.

11. Device according to any of claims 1 to 10, **characterised in that** said supply means (9) are integrated into the dressing.

12. Device according to any of claims 1 to 10, **characterised in that** said supply means (9) are designed to be removably mounted on the dressing.

13. Dressing usable in the production of a device for therapeutic treatment of wounds, consisting of an electrode (3) formed on a plane support (1), **characterised in that** said electrode (3) is covered with a dry hydrophile layer (8) which is not electrically conductive at the time it is applied to the wound.

14. Dressing according to claim 13, **characterised in that** the dry hydrophile layer (8) is a hydrocolloid mass.
